# EUROPEAN PATENT APPLICATION

(11) **EP 2 386 280 A1**
(43) Date of publication of application: **16.11.2011**
(21) Application number: 11164099.1
(22) Date of filing: 28.04.2011
(51) Int. Cl.: A61F 13/15

(54) **A disposable sanitary absorbent article assembly including an integrated disposal sheet and a method of using the assembly**

(30) Priority: 29.04.2010 US 769775; 29.04.2010 US 769793
(71) Applicant: Johnson & Johnson do Brasil Industria e Comercio Produtos Para Saude Ltda. Rodovia, San Jose dos Campos SP (BR)
(72) Inventor: Rainho das Neves, Rosana, Sao Jose dos Campos, Sao Paulo (BR)
(74) Representative: Kirsch, Susan Edith

(57) **Abstract**

A sanitary article assembly including a sanitary article and an integrated disposal sheet, the sanitary article assembly being structured and arranged to enable a user to manually separate the integrated disposal sheet from the article after the article has been soiled, roll the article from one end of the article to the opposite end of the article, and then enclose the article within the integrated disposal sheet by wrapping the integrated disposal sheet around the rolled article.

Also disclosed is a method of using a disposing of a sanitary article assembly including a sanitary article and an integrated disposal sheet, the sanitary article assembly being structured and arranged to enable a user to manually separate the Integrated disposal sheet from the article after the article has been soiled, roll the article from one end of the article to the opposite end of the article, and then enclose the article within the integrated disposal sheet by wrapping the integrated disposal sheet around the rolled article.

## Description

### FIELD OF THE INVENTION

The present invention generally relates to disposable sanitary absorbent articles and in particular to a disposable sanitary absorbent article including an integrated disposal sheet for disposing of the soiled article after use. The present invention also relates to a method of using such a sanitary absorbent article assembly.

### BACKGROUND OF THE INVENTION

Conventional sanitary absorbent articles such as sanitary napkins and the like are individually packaged prior to use to maintain the sanitary napkin clean prior to use. Often each individual sanitary napkin is folded in an overlapping configuration, commonly referred to as "tri-folded", and individually packaged in a polypropylene or polyethylene film pouch, commonly referred to as the "overwrap". In this manner, each individual napkin is maintained in a hygienic condition prior to use. When the user is ready to use the napkin, the napkin is removed from the "overwrap", unfolded, and arranged in the undergarment for use. A problem with above described overwrap structure is that once the napkin has been used (i.e. soiled) there is no effective way for the user to dispose of the soiled absorbent article in a hygienic manner. Rather, the user is often forced to simply place the soiled absorbent article in a refuse container or in the alternative the user may first wrap the article in toilet paper or the like prior to disposal.

In view of the foregoing, the inventors have recognized a need to provide a disposable sanitary absorbent article assembly including an integrated disposal sheet that enables the user to easily and discretely dispose of a soiled sanitary article after use.

### SUMMARY OF THE INVENTION

In view of the foregoing, the present invention provides, according to a first aspect of the invention, a sanitary article assembly including a sanitary article having a first transversely extending distal edge, a second transversely extending distal edge, a first longitudinally extending side edge, and a second longitudinally extending side edge, an integrated disposal sheet, wherein the integrated disposal sheet is fixedly attached to the sanitary article along the first transversely extending distal edge of the article and is manually separable from the sanitary article along the second transversely extending distal edge and the first and second longitudinally extending side edges of the article, and wherein the sanitary article assembly is structured and arranged to enable a user to manually separate the integrated disposal sheet from the sanitary article after the article has been soiled and roll the article from the second transversely extending distal edge to the first transversely extending distal edge in a first rotational direction and then enclose the article within the integrated disposal sheet by wrapping the integrated disposal sheet around the rolled article in a second rotational direction.

According to a second aspect of the invention, the present invention provides a sanitary article assembly including a sanitary article having a first and second transversely extending distal edge, a first and second longitudinally extending side edge; a liquid-permeable cover layer having a body-facing surface, a liquid-impermeable barrier layer having an inner surface and an outer surface, an integrated disposal sheet having an inner surface and a garment-facing surface, the garment facing surface having a positioning adhesive arranged thereon, wherein the integrated disposal sheet is arranged such that the inner surface of the integrated disposal sheet is arranged in face to face abutment with the outer surface of the barrier layer, wherein the integrated disposal sheet is fixedly attached to the barrier layer along the first transversely extending distal edge and the integrated disposal sheet is manually separable from the barrier layer along the second transversely extending distal edge and the first and second longitudinally side edges of the sanitary article, and wherein the sanitary article assembly is structured and arranged to enable a user to manually separate the integrated disposal sheet from the barrier layer after the article has been soiled and roll the article from the second transversely extending distal edge to the first transversely extending distal edge in a first rotational direction and then enclose the article within the integrated disposal sheet by wrapping the integrated disposal sheet around the rolled article in a second rotational direction.

The invention further provides a method of using a sanitary article assembly including the steps of arranging a sanitary article assembly in a crotch portion of an undergarment, removing the sanitary article assembly from the crotch portion after a sanitary article forming part of the assembly has been soiled, manually separating an integrated disposal sheet from the sanitary article, rolling the sanitary article from a second transversely extending distal edge of the sanitary article to a first transversely extending distal edge of the sanitary article in a first rotational direction and then enclosing the article within the integrated disposal sheet by wrapping the integrated disposal sheet around the rolled article in a second rotational direction.

### BRIEF DESCRIPTION OF THE DRAWINGS

Examples of embodiments of the present invention will now be described with reference to the drawings, in which:
Fig. 1 is a top perspective view of a sanitary article assembly according to the preset invention;
Fig. 2 is a sectional view taken along line 2-2 in Fig. 1;
Fig. 3 is a sectional view taken along line 3-3 in Fig. 1;
Fig. 4 is a partially exploded perspective view of the sanitary article assembly according to the present invention;
Fig. 5 is a bottom perspective view of a sanitary article assembly according to the present invention, showing the release paper thereof partially removed;
Fig. 6 depicts the manner in which the sanitary article assembly is applied to the crotch portion of an undergarment;
Fig. 7 depicts the manner in which the integrated disposal sheet is manually separable from the sanitary napkin that forms part of the sanitary article assembly;
Fig. 8 depicts the sanitary article assembly after the integrated disposal sheet has been arranged in its fully deployed position;
Fig. 9 is a sectional view take along line 9-9 in Fig. 8;
Figs. 10 and 11 depict the manner in which a user may roll the napkin from its second transversely extending distal edge to its first transversely extending distal edge in a first rotational direction; and
Figs. 12-15 depict the manner in which a user may enclose the rolled napkin within the integrated disposal sheet by wrapping the integrated disposal sheet around the rolled napkin in a second rotational direction.

### DETAILED DESCRIPTION OF THE INVENTION

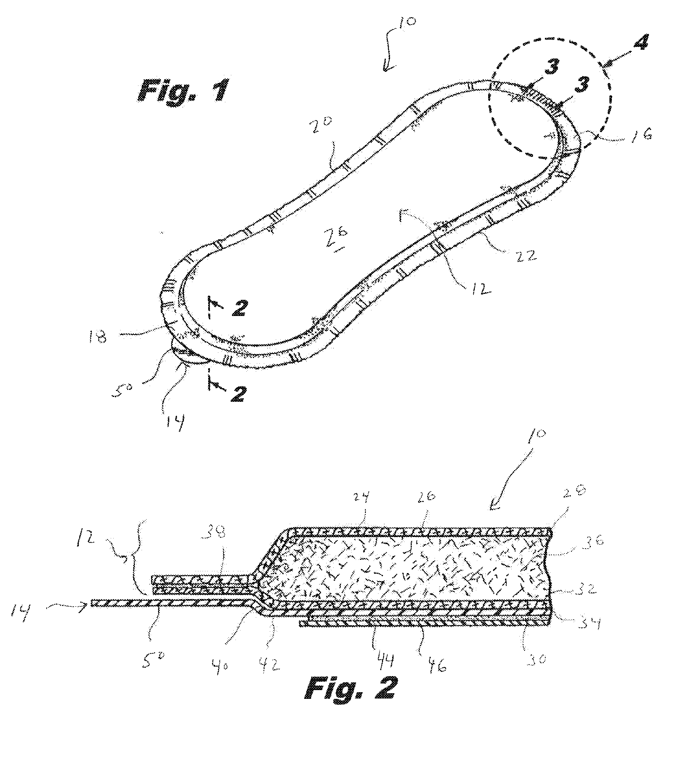

Referring to Figs. 1-5 the sanitary article assembly 10 according to the present invention generally includes a sanitary napkin 12 and an integrated disposal sheet 14.

Although the invention will be described herein with reference to a sanitary napkin 12, the invention may be utilized with other disposable sanitary absorbent articles such as adult incontinence products, panty liners, diapers and the like.

As shown in Fig. 1, the sanitary napkin 12 generally includes a first transversely extending distal edge 16, an opposed second transversely extending distal edge 18, a first longitudinally extending side edge 20, and an opposed second longitudinally extending side edge 22.

As best seen in Fig. 2, the sanitary napkin 12 includes a liquid-permeable cover layer 24 having a body facing surface 26 and an inner surface 28. The sanitary napkin 12 further includes a liquid-impermeable barrier layer 30 including an inner surface 32 and an outer surface 34. The sanitary napkin 12 also includes an absorbent core 36 arranged between the cover layer 24 and the barrier layer 30. Although not depicted in the Figures, the sanitary napkin 12 may optionally include a transfer layer arranged between the cover layer 24 and the absorbent core 36.

In one preferred embodiment of the invention the cover layer 24 is joined to the barrier layer 30 in areas adjacent their marginal edges by means of construction adhesive 38 arranged between the layers 24 and 30, as best seen in Fig. 2. In this manner the marginal portions of the cover layer 24 and the barrier layer 30 form an enclosure or flange seal that maintains the absorbent core 36 captive. Any suitable construction adhesive, well known to those of skill in the art, may be used to join the cover layer 24 to the barrier layer 30. The joint may alternatively be made by means of heat-bonding, ultrasonic bonding, radio frequency scaling, mechanical crimping, and the like and combinations thereof.

As shown in Fig. 2, the integrated disposal sheet 14 includes an inner surface 40 and a garment facing surface 42. As shown in Fig. 2 the integrated disposal sheet 14 is arranged such that the inner surface 40 thereof is arranged in abutting face to face relationship to the outer surface 34 of the liquid impermeable barrier layer 30. The garment facing surface 42 of the integrated disposal sheet 14 is preferably provided with a positioning adhesive 44 for securing the sanitary article assembly 10 to an undergarment during use. The positioning adhesive 44 may be covered with a removable release paper 46 so that the positioning adhesive is protected by the removable release paper prior to use. The release paper 46 can be formed from any suitable sheet-like material that adheres with sufficient tenacity to the adhesive to remain in place prior to use but which can be readily removed when the absorbent article is to be used. Optionally, a coating may be applied to release paper 46 to improve the ease of removabilty of the release paper from the adhesive. Any coating capable of achieving this result may be used, e.g., silicone.

The positioning adhesive may comprise a pressure sensitive adhesive and may be applied as strips, swirls, or waves, and the like. As used herein, the term pressure-sensitive adhesive refers to any releasable adhesive or releasable tenacious means. Suitable adhesive compositions, include, for example, water-based pressure-sensitive adhesives such as acrylate adhesives. Alternatively, the adhesive composition may include adhesives based on the following: emulsion or solvent-borne adhesives of natural or synthetic polyisoprene, styrene-butadiene, or polyacrylate, vinyl acetate copolymer or combinations thereof; hot melt adhesives based on suitable block copoylmers - suitable block copolymers for use in the invention include linear or radial co-polymer structures having the formula (A-B)x wherein block A is a polyvinylarene block, block B is a poly(monoalkenyl) block, x denotes the number of polymeric arms, and wherein x is an integer greater than or equal to one. Suitable block A polyvinylarenes include, but are not limited to Polystyrene, Polyalpha-methylstyrene, Polyvinyltoluene, and combinations thereof. Suitable Block B poly(monoalkenyl) blocks include, but are not limited to conjugated diene elastomers such as for example polybutadiene or polyisoprene or hydrogenated elastomers such as ethylene butylene or ethylene propylene or polyisobutylene, or combinations thereof. Commercial examples of these types of block copolymers include KratonTM elastomers from Shell Chemical Company, VectorTM elastomers from Dexco, SolpreneTM from Enichem Elastomers and StereonTM from Firestone Tire & Rubber Co.; hot melt adhesive based on olefin polymers and copolymers where in the olefin polymer is a terpolymer of ethylene and a co-monomers, such as vinyl acetate, acrylic acid, methacrylic acid, ethyl acrylate, methyl acrylate, n-butyl acrylate vinyl silane or maleic anhydride. Commercial examples of these types of polymers include Ateva( polymers from AT plastics), Nucrel( polymers from DuPont), Escor (from Exxon Chemical).

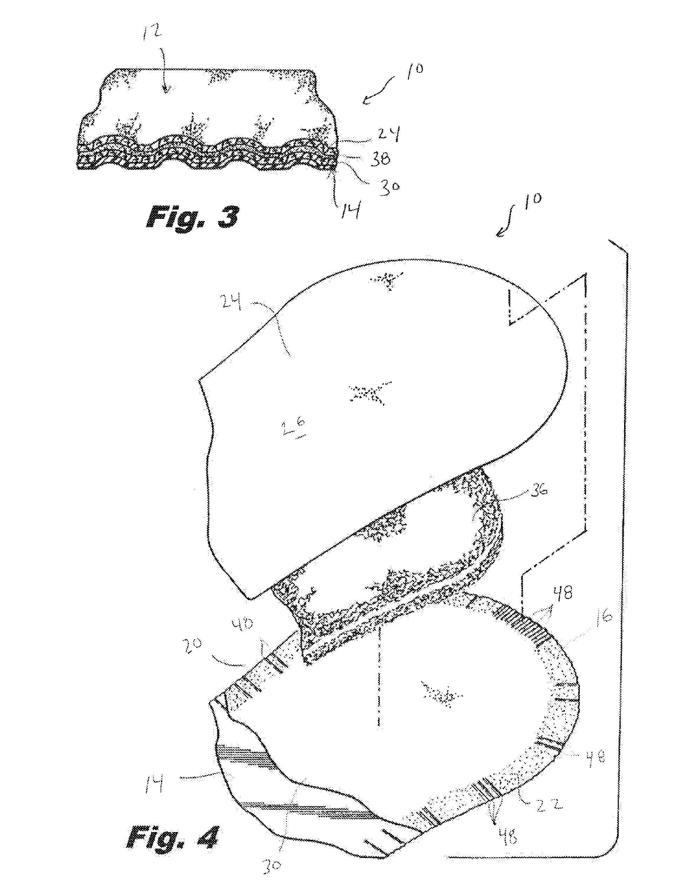

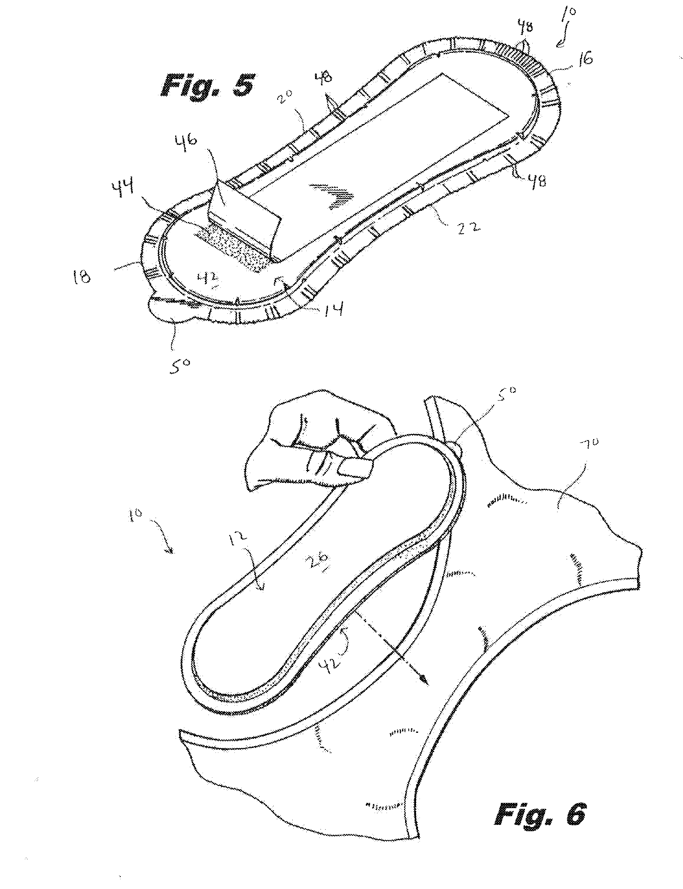

As shown in Figs. 4 and 5, the integrated disposal sheet 14 is preferably attached to the barrier layer 30 by means of a plurality of individual crimps 48 formed in zones located adjacent the marginal edges of the sheet 14 and barrier layer 30. As shown in Figs. 4 and 5, there are fewer crimps 48 on a per unit length basis located adjacent the second transversely extending distal edge 18 and adjacent the first and second longitudinally extending edges 20 and 22, as compared to the number of crimps 48 located adjacent the first transversely extending distal edge 16. Stated another way, the crimps 48 are more densely arranged adjacent the first transversely extending distal edge 16. As will be described in further detail below, the above described structure enables the integrated disposal 14 sheet to remain securely attached to the barrier layer 30 during use of the sanitary article assembly 10 (i.e. while the assembly is arranged in the undergarment of the user) but enables the user to manually separate the integrated disposal sheet 14 from the barrier layer 30 along the second transversely extending distal edge 18 and the first and second longitudinally extending side edges 20, 22 of the sanitary napkin 12. It is noted that, due to the more dense arrangement of the crimps 48 adjacent first transversely extending distal edge 16, the disposal sheet 14 remains permanently secured to the barrier layer 30 adjacent the first transversely extending distal edge 16 at all times. Preferably, during manufacture, the integrated disposal sheet 14 is secured to the barrier layer 30 prior to the attachment of the barrier layer 30 to the cover layer 24 as described above.

The individual crimps 48 may be formed by pressure and/or heat and pressure as will be understood to those of skill in the art. The strength of the individual crimps should be selected to enable the integrated disposal sheet 14 to remain securely attached to the barrier layer 30 during use of the sanitary article assembly 10 (i.e. while the assembly 10 is arranged in the user's undergarment) while at the same time permit enable the user to manually separate the integrated disposal sheet 14 from the barrier layer 30 as described above.

In lieu of the crimps 48 described above, adhesive could be used to selectively secure the disposal sheet 14 to the barrier 30 by applying adhesive in a similar pattern to the crimp pattern shown in Figs. 4 and 5. Alternatively, two adhesives could be employed, for example a less aggressive adhesive could be arranged adjacent the second transversely extending distal edge 18 and the first and second longitudinally extending side edges 20, 22, and a more aggressive adhesive could be arranged adjacent first transversely extending distal edge 16.

If adhesive(s) is used to selectively secure the disposal sheet 14 to the barrier 30, such adhesive is preferably a hot melt adhesive based on styrenic block copolymers, that is the adhesive formulation contains styrenic block copolymers, tackifying resins, and plasticizing oils. More specifically, the adhesive is preferably made of styrene-isoprene-styrene block copolymers (SIS) or styerene-ethylene-butylene block copolymers (SEBS).

Suitable block copolymers for use in the invention include linear or radial co-polymer structures having the formula (A-B)x wherein block A is a polyvinylarene block, block B is a poly(monoalkenyl) block, x denotes the number of polymeric arms, and wherein x is an integer greater than or equal to one. Suitable block A polyvinylarenes include, but are not limited to Polystyrene, Polyalpha- methylstyrene, Polyvinyltoluene, and combinations thereof. Suitable Block B poly(monoalkenyl) blocks include, but are not limited to conjugated diene elastomers such as for example polybutadiene or polyisoprene or hydrogenated elastomers such as ethylene butylene or ethylene propylene or polyisobutylene, or combinations thereof. Commercial examples of these types of block copolymers include Kraton elastomers from Kraton Polymers LLC, Vector elastomers from Dexco polymers, Stereon from Firestone Tire & Rubber Co. & SIBStar Polymers from Kaneka Co. Ltd.

Suitable tackifying resins include aliphatic petroleum resins and the hydrogenated derivatives thereof; aromatic petroleum resin and the hydrogenated derivatives thereof; and aliphatic or aromatic petroleum resins and the hydrogenated derivatives thereof, and combinations thereof; natural and modified resins; glycerol and pentaerythritol esters of natural and modified resins; polyterpene resins; copolymers and terpolymers of natural terpenes; phenolic modified terpene resins and the hydrogenated derivatives thereof. Commercial examples of these types of resins include Escorez from Exxon Chemical aliphatic hydrocarbon and cycloaliphatic resins, Wingtacke from Goodyear Tire & Rubber Co. synthetic polyterpene resins including aromatic modified versions, Arkon partially and fully hydrogenated aromatic resins from Arakawa Chemicals,Foral hydrogenated rosin ester, Staybelite hydrogenated modified rosin, Poly-pale polymerized rosin, Permalyn rosin ester, Pentalyn rosin ester, Adtac oil extended hydrocarbon resin, Piccopale aromatic hydrocarbon, Piccotac, Hercotac aromatic modified aliphatic hydrocarbon, is Regalrez cycloaliphatic resins, or Piccolyte from Eastman Chemical Co., Zonatac styrenated terpene resin, Zonarez rosin ester and Zonester rosin ester from Arizona Chemical and Nevtac aromatic modified aliphatic hydrocarbon from Neville Chemical Company.

Specific commercially available adhesives that may be used to selectively secure the disposal sheet 14 to the barrier 30 include HM-2703, HL-2268, and HL-2110X from HB Fuller Co. of St. Paul, Minnesota.

If more than one adhesive is used to selectively secure the disposal sheet 14 to the barrier 30 (e.g. for example a less aggressive adhesive is arranged adjacent the second transversely extending distal edge 18 and the first and second longitudinally extending side edges 20, 22, and a more aggressive adhesive is arranged adjacent first transversely extending distal edge 16), then one of the adhesives described above could be employed as the less aggressive adhesive and a conventional construction adhesive, such as NW-1023 from HB Fuller Co., could be employed as the more aggressive adhesive.

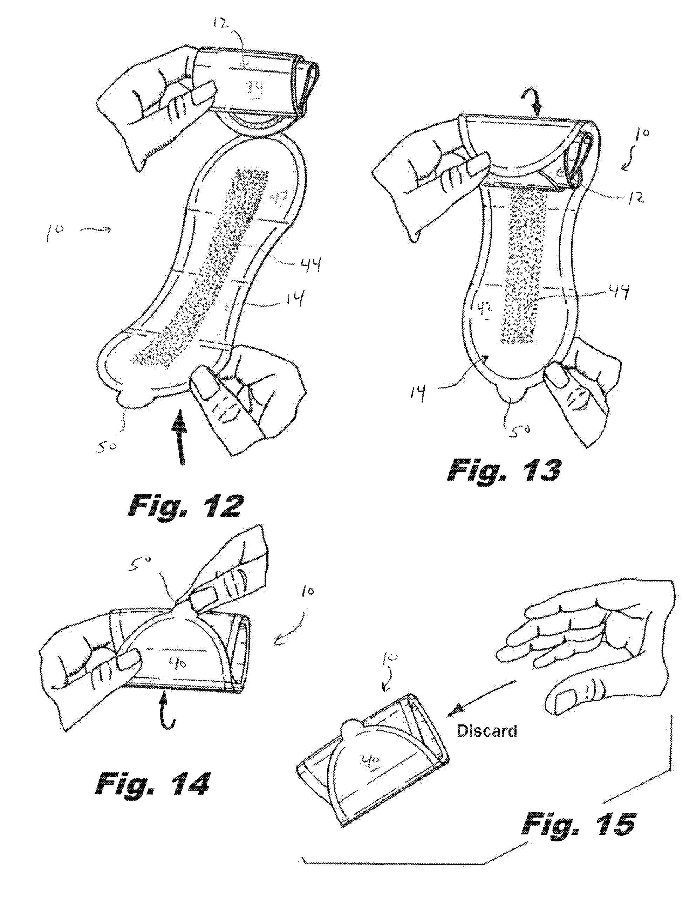

Preferably, the adhesive(s) used to selectively secure the disposal sheet 14 to the barrier 30 is selected so that the adhesive(s) has more chemical affinity with the outer surface 34, of the liquid-impermeable barrier layer 30 than with the inner surface 40, of the integrated disposal sheet 14. In this way, when the assembly 10 is rolled for disposal (as shown in Fig. 15), the surface 40 retains minimal traces of adhesive residue. To provide this benefit, the liquid-impermeable barrier layer 30 and the integrated disposal sheet 14 may be constructed from different materials so that the material that forms the liquid-impermeable barrier layer 30 has more chemical affinity with the adhesive than the material that forms integrated disposal sheet 14. Alternatively, the liquid-impermeable barrier layer 30 and the disposal sheet 14 could be formed from the same material, and the desired chemical affinity could be provided by coating the outer surface 34 or the inner surface 40 with any selected material to increase or reduce the chemical affinity of material to the adhesive. Other means to provide the desired chemical affinity will also be apparent to those of skill in the art

Other means to secure the integrated disposal sheet 14 to the barrier layer 30 that enable the integrated disposal sheet 14 to be manually separated from the barrier layer 30 will be apparent to those of skill in the art and are thus are considered within the scope of the present invention.

As shown in Figs. 4 and 5, the integrated disposal sheet 14 has the same general shape as the barrier layer 30 and is substantially coextensive therewith. The integrated disposal sheet 14 may be provided with a tab 50 that preferably extends beyond the second transversely extending distal edge 18 of the sanitary napkin 12. As will be described in greater detail below, the tab 50 enables a user to more easily grasp the integrated disposal sheet 14 and separate the same from the barrier layer 30.

A method of using the absorbent article assembly 10 according to the present invention will now be described with reference to Figures 5-15. As shown in Fig. 5, a user first removes the release paper 46 from the sanitary article assembly 10 to thereby expose the positioning adhesive 44 arranged on the garment facing surface 42 of the integrated disposal sheet 14. Thereafter, as shown in Fig. 6, the user can arrange the absorbent article assembly 10 in the crotch portion of an undergarment 70. Specifically, the user places the absorbent article assembly 10 such that garment facing surface 42 of the integrated disposal sheet 14 is arranged in abutting contact with the crotch portion of the undergarment. In this manner, the positioning adhesive 44 secures the assembly 10 to the undergarment during use.

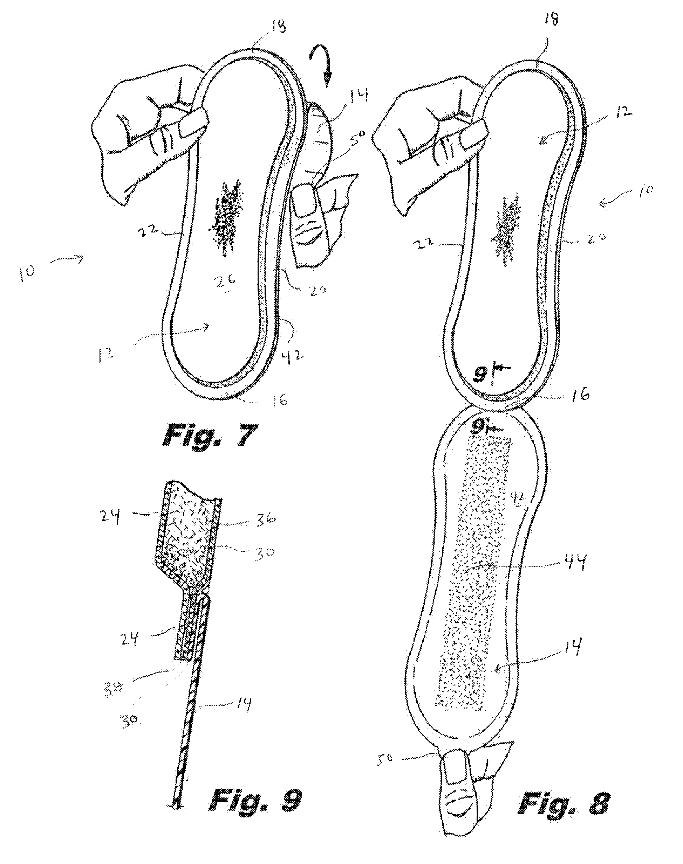

Once the sanitary napkin 12 has been soiled the user may remove the assembly 10 from the undergarment. Thereafter, as shown in Fig. 7, the user can grasp the integrated disposal sheet 14 by the tab 50 and begin to separate integrated disposal sheet 14 from the barrier layer 30 starting from the second transversely extending distal edge 18 of the sanitary napkin 12. As the user continues to pull the integrated disposal sheet 14, the sheet 14 separates from the barrier layer 30 along the first and second longitudinally extending side edges 22 and 20 until the disposal sheet 14 is only secured to the barrier layer 30 adjacent the first transversely extending distal edge 16, as shown in Figs. 8 and 9.

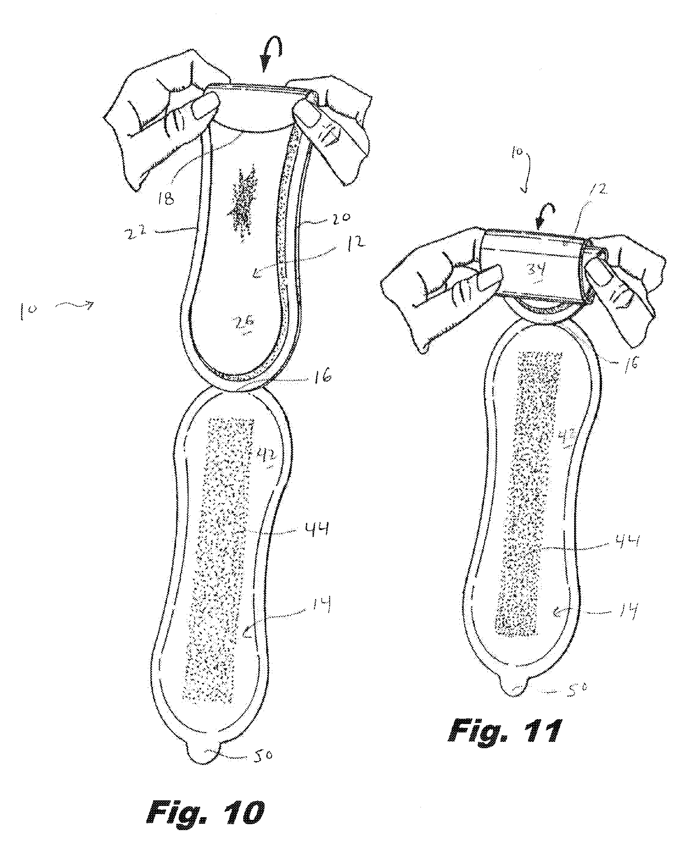

Once the integrated disposal sheet 14 has been fully deployed, as shown in Fig. 10, the user may then begin to roll the sanitary napkin 12 from the second transversely extending distal edge 18 towards the first transversely extending distal edge 16. It is noted that the sanitary napkin 12 is rolled in a first rotational direction, specifically in the orientation depicted in the Figures, the napkin 12 is rolled in a direction out of the page. As shown in Fig. 11, the user continues to roll the napkin 12 until the user reaches the first transversely extending distal edge 16.

Once the napkin 12 has been fully rolled, as shown in Fig. 12, the user may then grasp the deployed integrated disposal sheet 14 and begin to wrap the integrated disposal sheet 14 around the rolled napkin 12. It is noted that the integrated disposal sheet 14 is wrapped around the rolled napkin 12 in a second rotational direction that is opposite to the first rotational direction. Thus, in the orientation depicted in the Figures, the integrated disposal sheet 14 is wrapped around the rolled napkin 12 in a direction that is into the page. It is further noted that as the user wraps the integrated disposal sheet 14 around the rolled napkin 12 the integrated disposal sheet 14 is retained in place against the outer surface 34 of the barrier layer 30 by means of the positioning adhesive 44 that is located on the garment facing surface 42 of the disposal sheet 14. As shown in Figs. 13-15, the user continues to wrap the disposal sheet 14 around the rolled napkin 12 until the napkin 12 is fully enclosed within the integrated disposal sheet 14 as shown in Fig. 15. Thereafter, the user may conveniently and discretely dispose of the assembly 10.

### Cover Layer

The cover layer 24 may be a relatively low density, bulky, high-loft non-woven web material. The cover layer 24 may be composed of only one type of fiber, such as polyester or polypropylene or it may include a mixture of more than one fiber. The cover may be composed of bi-component or conjugate fibers having a low melting point component and a high melting point component. The fibers may be selected from a variety of natural and synthetic materials such as nylon, polyester, rayon (in combination with other fibers), cotton, acrylic fiber and the like and combinations thereof.

Bi-component fibers may be made up of a polyester layer and a polyethylene sheath. The use of appropriate bi-component materials results in a fusible non-woven fabric. Examples of such fusible fabrics are described in U.S. Pat. No. 4,555,430 issued Nov. 26, 1985 to Chicopee. Using a fusible fabric increases the ease with which the cover layer may be mounted to the absorbent layer and/or to the barrier layer.

The cover layer 24 preferably has a relatively high degree of wettability, although the individual fibers comprising the cover may not be particularly hydrophilic. The cover material should also contain a great number of relatively large pores. This is because the cover layer 24 is intended to take-up body fluid rapidly and transports it away from the body and the point of deposition. Therefore, the cover layer contributes little to the time taken for the napkin to absorb a given quantity of liquid (penetration time).

Advantageously, the fibers that make up the cover layer 24 should not lose there physical properties when they are wetted, in other words they should not collapse or lose their resiliency when subjected to water or body fluid. The cover layer 24 may be treated to allow fluid to pass through it readily. The cover layer 24 also functions to transfer the fluid quickly to the underlying layers of the absorbent article. Thus, the cover layer 24 is advantageously wettable, hydrophilic and porous. When composed of synthetic hydrophobic fibers such as polyester or bi-component fibers, the cover layer 24 may be treated with a surfactant to impart the desired degree of wettability.

Alternatively, the cover layer 24 can also be made of polymer film having large pores. Because of such high porosity, the film accomplishes the function of quickly transferring body fluid to the underlying layers of the absorbent article.

The cover layer 24 may be embossed to the absorbent core 36 in order to aid in promoting hydrophilicity by fusing the cover to the next layer. Such fusion may be effected locally, at a plurality of sites, or over the entire contact surface of cover layer 24 and absorbent core 36.

### Transfer Layer

Adjacent to the cover layer 24 on its inner side the sanitary napkin 12 may be provided with an optional transfer layer (not shown in the drawings). The transfer layer provides the means of receiving body fluid from the cover layer 24 and holding it until the underlying absorbent core 36 has an opportunity to absorb the fluid, and therefore acts as a fluid transfer or acquisition layer.

The transfer layer is, preferably, more dense than and has a larger proportion of smaller pores than the cover layer 24. These attributes allow the transfer layer to contain body fluid and hold it away from the outer side of the cover layer 24, thereby preventing the fluid from rewetting the cover layer 24 and its surface. However, the transfer layer is, preferably, not so dense as to prevent the passage of the fluid through the layer into the underlying absorbent core 36.

The transfer layer may be composed of fibrous materials, such as wood pulp, polyester, rayon, flexible foam, or the like, or combinations thereof. The transfer layer may also comprise thermoplastic fibers for the purpose of stabilizing the layer and maintaining its structural integrity. The transfer layer may be treated with surfactant on one or both sides in order to increase its wettability, although generally the transfer layer is relatively hydrophilic and may not require treatment. The transfer layer is preferably bonded or adhered on both sides to the adjacent layers, i.e. the cover layer 24 and the underlying absorbent core 36.

Examples of suitable materials for the transfer layer include through air bonded pulp sold by Buckeye of Memphis, Tenn., under the designation VIZORB 3008, VIZORB 3042, and VIZORB 3010.

### Absorbent Core

In one preferred embodiment of the invention, the absorbent core 36 is a blend or mixture of cellulosic fibers and superabsorbent disposed therein. Cellulosic fibers that can be used in the absorbent core 36 are well known in the art and include wood pulp, cotton, flax and peat moss. Wood pulp is preferred. Pulps can be obtained from mechanical or chemi-mechanical, sulfite, kraft, pulping reject materials, organic solvent pulps, etc. Both softwood and hardwood species are useful. Softwood pulps are preferred. It is not necessary to treat cellulosic fibers with chemical debonding agents, cross-linking agents and the like for use in the present material. Some portion of the pulp may be chemically treated as discussed in U.S. Pat. No. 5,916,670 to improve flexibility of the product. The flexibility of the material may also be improved by mechanically working the material or tenderizing the material.

The absorbent core 36 can contain any superabsorbent polymer (SAP), which are well known in the art. For the purposes of the present invention, the term "superabsorbent polymer" (or "SAP") refers to materials, which are capable of absorbing and retaining at least about 10 times their weight in body fluids under a 0.5 psi pressure. The superabsorbent polymer particles of the invention may be inorganic or organic crosslinked hydrophilic polymers, such as polyvinyl alcohols, polyethylene oxides, crosslinked starches, guar gum, xanthan gum, and the like. The particles may be in the form of a powder, grains, granules, or fibers. Preferred superabsorbent polymer particles for use in the present invention are crosslinked polyacrylates, such as the product offered by Sumitomo Seika Chemicals Co., Ltd. Of Osaka, Japan, under the designation of SA70N and products offered by Stockhausen Inc..

### Barrier Layer

Underlying the absorbent core 36 is a barrier layer 30 comprising a liquid-impervious film material so as to prevent liquid that is entrapped in the absorbent core 36 from egressing the sanitary napkin and staining the wearer's undergarment. The barrier layer 30 is preferably made of polymeric film, although it may be made of liquid impervious, air-permeable material such as repellent-treated non-woven or micropore films or foams. Suitable commercially available barrier materials include polyethylene barrier materials of the type available from Clopay do Brasil, São Paulo, SP, Brazil,

The barrier layer may be breathable, i.e., permits vapor to transpire. Known materials for this purpose include nonwoven materials and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet.

### Integrated Disposal Sheet

The integrated disposal sheet 14 may be constructed from the same or similar compatible materials as those described above with respect to the barrier layer. Selection of such a compatible material readily allows the integrated disposal sheet 14 to be crimp sealed to the barrier layer 30 in the manner described herein above. Suitable commercially available materials include polyethylene materials of the type available from Clopay do Brasil, São Paulo, SP, Brazil.

Alternatively, the integrated disposal sheet 14 may be constructed from a tissue material such as Little Rapids type 2004 wetlaid tissue commercially available from Little Rapids Corp., Green Bay, Wisconsin. If such a tissue material is employ then the tissue material is preferably secured to the barrier layer 30 by means of adhesive as described above. The use of a tissue like material enables the product to be camouflaged in the trash can among toilet tissue paper, after disposal.

Variations of the sanitary article assembly according to the present invention may be apparent to those of skill in the art based upon the disclosure of the present application. Thus, it is intended that the present application cover the modifications and variations of this invention provided that they come within the scope of the appended claims and their equivalents.

## Claims

**1.** A sanitary article assembly comprising:
a sanitary article having a first transversely extending distal edge, a second transversely extending distal edge, a first longitudinally extending side edge, and a second longitudinally extending side edge;
an integrated disposal sheet;
wherein the integrated disposal sheet is fixedly attached to the sanitary article along the first transversely extending distal edge of the article and is manually separable from the sanitary article along the second transversely extending distal edge and the first and second longitudinally extending side edges of the article; and
wherein the sanitary article assembly is structured and arranged to enable a user to manually separate the integrated disposal sheet from the sanitary article after the article has been soiled and roll the article from the second transversely extending distal edge to the first transversely extending distal edge in a first rotational direction and then enclose the article within the integrated disposal sheet by wrapping the integrated disposal sheet around the rolled article in a second rotational direction.

**2.** The sanitary article assembly according to claim 1, wherein the sanitary article includes a liquid permeable cover layer having a body-facing surface, a liquid impermeable barrier layer having an inner surface and an outer surface.

**3.** The sanitary article assembly according to claim 1 or claim 2, wherein the integrated disposal sheet has an inner surface and a garment facing surface.

**4.** The sanitary article assembly according to claim 3, further comprising a positioning adhesive arranged on the garment facing surface.
The sanitary article assembly according to claim 3 or claim 4, wherein the integrated disposal sheet is fixedly attached to the barrier layer along the first transversely extending distal edge and the integrated disposal sheet is manually separable from the barrier layer along the second transversely extending distal edge and the first and second longitudinally side edges of the sanitary article.

**6.** The sanitary article assembly according to claim 5, wherein prior to the manual separation of the integrated disposal sheet from the barrier layer, the integrated disposal sheet is arranged such that an inner surface of the integrated disposal sheet is arranged in face to face abutment with the outer surface of the barrier layer.

**7.** The sanitary article assembly according to any one of claims 2 to 6, wherein the sanitary article further comprises:
an absorbent core arranged between the cover layer and the barrier layer.

**8.** The sanitary article assembly according to any preceding claim, wherein the sanitary article is one of a sanitary napkin, a panty liner, diaper, and adult incontinence product.

**9.** The sanitary article assembly according to any preceding claim, wherein the integrated disposal sheet includes a tab that extends beyond the second transversely extending distal edge of the sanitary article.

**10.** The sanitary article assembly according to any preceding claim, wherein the integrated disposal sheet is attached to the sanitary article by means of a plurality of individual crimps.

**11.** The sanitary article assembly according to claim 10, wherein the plurality of crimps are arranged adjacent to the first and second transversely extending distal edge and the first and second longitudinally extending side edge.

**12.** The sanitary article assembly according to claim 11, wherein the plurality of crimps are more densely arranged adjacent the first transversely extending distal edge relative to the crimps located adjacent the second transversely extending distal edge the first and second longitudinally extending side edge.

**13.** A sanitary article assembly according to claim 1 additionally comprising:
a liquid-permeable cover layer having a body-facing surface; and
a liquid-impermeable barrier layer having an inner surface and an outer surface;
and wherein the integrated disposal sheet has an inner surface and a garment-facing surface, the garment facing surface having a positioning adhesive arranged thereon;
wherein the integrated disposal sheet is arranged such that the inner surface of the integrated disposal sheet is arranged in face to face abutment with the outer surface of the barrier layer; and
wherein the integrated disposal sheet is fixedly attached to the barrier layer along the first transversely extending distal edge and the integrated disposal sheet is manually separable from the barrier layer along the second transversely extending distal edge and the first and second longitudinally side edges of the sanitary article,

**14.** A method of using a sanitary article assembly comprising the steps of:
arranging a sanitary article assembly in a crotch portion of an undergarment;
removing the sanitary article assembly from the crotch portion after a sanitary article forming part of the assembly has been soiled;
manually separating an integrated disposal sheet from the sanitary article;
rolling the sanitary article from a second transversely extending distal edge of the sanitary article to a first transversely extending distal edge of the sanitary article in a first rotational direction and then enclosing the article within the integrated disposal sheet by wrapping the integrated disposal sheet around the rolled article in a second rotational direction.

**15.** The method of claim 14, wherein the sanitary article assembly is as claimed in any one of claims 1 to 13,

**16.** The method according to claim 14 or claim 15, further comprising the step of:
prior to the arrangement of the sanitary article assembly in the crotch portion of the undergarment, removing a release paper from sanitary article assembly thereby expose a positioning adhesive arranged on a garment facing surface of the integrated disposal sheet.

**17.** The method according to claim 14, claim 15 or claim 16, wherein the step of manually separating the integrated disposal sheet from the sanitary article comprises the steps of:
grasping the integrated disposal sheet and manually separating the integrated disposal sheet from the sanitary article starting at the at a second transversely extending distal edge of the article and separating the integrated disposal sheet from the sanitary article along the first and second longitudinally extending side edges of the article.

**18.** The method according to claim 17, wherein the integrated disposal sheet is manually separated from the sanitary article along the second transversely extending distal edge, and the first and second longitudinally extending side edges of the sanitary article, until the integrated disposal sheet is only attached to the sanitary article along the first transversely extending distal edge of the sanitary article.

**19.** The method according to claim 18, further comprising:
retaining the integrated disposal sheet against the rolled sanitary article by means of the positioning adhesive located on the garment facing surface of the disposal sheet.

**20.** The method according to claim 19, further comprising the step of:
disposing of the sanitary article assembly with sanitary article enclosed within the disposal sheet in a rolled state.
